(19) **Europäisches Patentamt / European Patent Office / Office européen des brevets**

(11) **EP 3 853 653 B1**

(12) **FASCICULE DE BREVET EUROPEEN**

(45) Date de publication et mention de la délivrance du brevet:
**05.10.2022 Bulletin 2022/40**

(21) Numéro de dépôt: **19790655.5**

(22) Date de dépôt: **17.09.2019**

(51) Classification Internationale des Brevets (IPC):
*G02B 26/00* *(2006.01)*    *G01J 1/00* *(2006.01)*
*G01J 9/00* *(2006.01)*    *H04N 5/374* *(2011.01)*
*A61B 3/10* *(2006.01)*    *A61B 3/14* *(2006.01)*
*H04N 5/232* *(2006.01)*

(52) Classification Coopérative des Brevets (CPC):
**H04N 5/374; H04N 5/23229;** A61B 3/1015;
G01J 2009/002

(86) Numéro de dépôt international:
**PCT/FR2019/052160**

(87) Numéro de publication internationale:
**WO 2020/058623 (26.03.2020 Gazette 2020/13)**

(54) **PROCÉDÉ, SYSTÈME ET PROGRAMME D'ORDINATEUR ADAPTATIVES D'ACQUSITION D'UNE IMAGE**

ADAPTIVE VERFAHREN, SYSTEM UND COMPUTERPROGRAMM ZUR AUFNAHME EINES BILDES

ADAPTIVE METHOD, SYSTEM AND COMPUTER PROGRAM FOR CAPTURING AN IMAGE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.09.2018 FR 1858510**

(43) Date de publication de la demande:
**28.07.2021 Bulletin 2021/30**

(73) Titulaires:
- **CENTRE NATIONAL DE LA RECHERCHE SCIENTIFIQUE (CNRS)**
  **75016 Paris (FR)**
- **SORBONNE UNIVERSITE**
  **75006 Paris (FR)**
- **Ecole Normale Supérieure**
  **75005 Paris (FR)**
- **Université Paris Cité**
  **75006 Paris (FR)**
- **Université de Bourgogne**
  **21000 Dijon (FR)**
- **Observatoire de Paris**
  **75014 Paris (FR)**

(72) Inventeurs:
- **DARSON, David**
  **77000 Vaux Le Penil (FR)**
- **DUBOIS, Julien**
  **21800 Quetigny (FR)**
- **COLAS, François**
  **75014 Paris (FR)**

(74) Mandataire: **Regimbeau**
**20, rue de Chazelles**
**75847 Paris Cedex 17 (FR)**

(56) Documents cités:
**US-A1- 2005 098 707    US-A1- 2013 250 240
US-A1- 2014 270 565**

EP 3 853 653 B1

## Description

CONTEXTE ET ETAT DE LA TECHNIQUE

La présente invention se rapporte au domaine de l'acquisition d'une image par un imageur. Plus précisément, l'invention concerne un procédé d'acquisition d'une image par un imageur exposé à un flux lumineux incident traversant le chemin optique d'un système optique comprenant un organe de correction de front d'onde, afin de mettre en œuvre une technique d'optique adaptative.

Un procédé connu pour la correction de front d'onde est divulgué par la demande de brevet US 2005/0098707 A1.

La résolution d'un système optique est étroitement liée à sa capacité à séparer les plus fins détails d'un objet vu par un imageur. Dans un système optique parfait de diamètre d'ouverture D, la résolution est limitée, selon le critère de Rayleigh, par la diffraction, à un angle de vue minimum entre deux détails de $\Delta\phi=1.44\lambda/D$ (où $\lambda$ est la longueur d'onde). Dans la réalité, beaucoup d'éléments peuvent venir dégrader ce pouvoir séparateur théorique, ou encore la PSF (Point Spread Factor) connue également sous le nom de réponse impulsionnelle spatiale du système optique-imageur : en premier lieux, tous les défauts et aberrations optiques générés par le système optique lui-même mais également tous ceux issus du milieu de propagation de la lumière. Si les progrès techniques ont permis de réaliser des systèmes optiques pratiquement sans défauts ou aberrations statiques optiques intrinsèques, il n'est pas toujours possible de modifier les caractéristiques dynamiques que ce soit du système opto-mécanique lui-même ou bien du milieu de propagation.

Lors de son trajet depuis une source lumineuse jusqu'à un imageur, un flux lumineux subit des distorsions de son front d'onde en raison des perturbations du milieu de propagation, résultant notamment de l'inhomogénéité dudit milieu. En dehors de tous les défauts statiques, tous les défauts dynamiques du système opto-mécanique (dilatation-contraction, vibration, etc.) peuvent également dégrader la PSF de l'ensemble optique-imageur. PSF qui dans un système parfait, sans aucun défaut ni aberrations, n'est soumise qu'à la seule limite de diffraction du système considéré.

Par exemple, en astronomie, la lumière en provenance d'une étoile ponctuelle se propage en ligne droite, dans le vide, jusqu'à la Terre. Le front d'onde du flux lumineux est alors un plan et reste un plan dans le vide, en principe capable de redonner une tache limitée par diffraction dans un télescope qui recevrait cette lumière, ce qui est le cas des différents télescopes placés en orbite dans le vide spatial.

Toutefois, dans le cas d'un imageur tel qu'un télescope sur le sol de la Terre, le flux lumineux traverse également l'atmosphère terrestre. L'atmosphère est constituée de gaz dont la composition et surtout la densité varie dans le temps et l'espace, et présente donc des fluctuations d'indice de réfraction, dont résultent les turbulences atmosphériques subies par le flux lumineux.

De même, dans le domaine de l'imagerie oculaire, nos yeux sont constamment en mouvement ; même en fixation, nos yeux sont agités par des mouvements involontaires. Ces mouvements, dits de fixation, sont indispensables à la vision, car ils permettent de maintenir la perception visuelle. Ainsi, les milieux traversés par le flux lumineux sont en mouvement. De plus, le flux lumineux d'éclairage traverse plusieurs milieux (la cornée, le cristallin ou le corps vitré) présentant des compositions, des densités et des indices de réfaction variés, qui peuvent altérer le front d'onde. D'autres aberrations dynamiques peuvent également survenir, par exemple dues aux micro-accommodations du cristallin ou à l'écoulement du film lacrymal.

Il en résulte que dans la plupart des applications, les caractéristiques du milieu de propagation du flux lumineux varient en permanence. Cela se traduit par des distorsions dans le front d'onde du flux lumineux, qui varient également, et en particulier qui varient au cours de l'exposition de l'imageur en train d'acquérir une image. La taille de la tâche de diffraction au niveau de l'imageur augmente considérablement, dégradant la PSF et abaissant par conséquent la résolution de l'image acquise par cet imageur. Cet effet est d'autant plus accentué que l'exposition est prolongée durant l'acquisition d'une image.

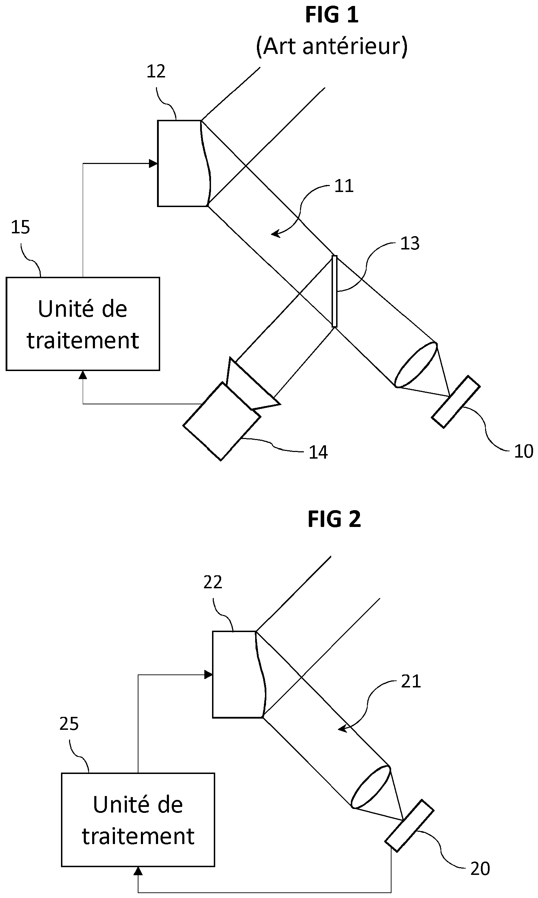

Pour améliorer la qualité des images acquises, il a été développé des solutions pour mesurer et compenser les distorsions du front d'onde du flux lumineux incident. L'une de ces solutions est l'optique adaptative, qui permet de corriger en temps réel les déformations évolutives et non-prédictives du front d'onde. La figure 1 montre un exemple typique d'un système optique de l'état de la technique mettant en œuvre une technique d'optique adaptative.

Le système comprend alors un imageur 10 recevant de la lumière par un chemin optique 11. Le chemin optique 11 comprend un organe de correction de front d'onde 12 (typiquement un miroir déformable) en aval d'une lame séparatrice 13. La lame séparatrice 13 dévie une partie du flux lumineux vers un dispositif de mesure du front d'onde 14 (un analyseur de front d'onde, par exemple un capteur type Shack-Hartmann). Une boucle de contrôle comprenant une unité de traitement de données 15 utilise la mesure du dispositif de mesure du front d'onde 14 pour déterminer la commande de l'organe de correction de front d'onde 13. En fonction de cette commande, la configuration de l'organe de correction de front d'onde 13 est modifiée afin de corriger le front d'onde du flux lumineux parcourant le chemin optique 11 avant son arrivée sur l'imageur 10.

Un tel système nécessite donc deux dispositifs de prise en vue : l'imageur 10 qui doit acquérir l'image, et le dispositif de mesure du front d'onde 14 qui doit mesurer les distorsions du front d'onde. Il en résulte une complexité du système et un encombrement important. De plus, les deux dispositifs de prise en vue étant spatialement distants, des contraintes mécaniques différentes peuvent apparaître en termes de perturbations auxquelles ces dispositifs sont soumis comme par exemple les vibrations.

Par ailleurs, la présence de la lame séparatrice 13 divisant le flux lumineux implique que l'imageur 10 ne reçoit qu'une partie de ce flux lumineux lorsqu'il acquiert une image, ce qui rend nécessaire un temps d'exposition plus important afin de compenser la déviation d'une partie du flux lumineux, augmentant les problèmes de variation du flux lumineux, voire peut empêcher d'imager certains objets peu lumineux, qui par exemple ne se distingueraient plus suffisamment du bruit de mesure.

Il a donc été développé des systèmes mettant en œuvre une optique adaptative sans utiliser de dispositif de mesure du front d'onde dédié : c'est l'imageur qui récolte les données utilisées pour analyser le front d'onde. Plus précisément, les premières images acquises par l'imageur sont utilisées pour déterminer la commande à appliquer à l'organe de correction de front d'onde 13. Ainsi, les premières images sont détruites afin de déterminer la correction à appliquer. Par ailleurs, cette approche suppose que les distorsions du front d'onde varient faiblement pendant l'acquisition d'une image, puisque c'est la correction déterminée à partir de l'image précédente qui est utilisée. Il est donc nécessaire pour la correction de rester dans le temps de cohérence de la turbulence considérée, ce qui implique d'augmenter suffisamment le rythme d'acquisition, en termes d'images par seconde, ce qui diminue d'autant le signal exploitable sur les images individuelles. Certains systèmes combinent l'imageur et le dispositif de mesure du front d'onde dans une même matrice. Il en résulte une perte de résolution des images acquises, alors même que l'optique adaptative vise à améliorer la résolution.

PRESENTATION DE L'INVENTION

L'invention a pour but de permettre l'acquisition d'une image présentant une résolution améliorée, et une augmentation du rapport signal sur bruit ou SNR ( de l'anglais "Signal to Noise Ratio") consécutive à l'amélioration de la PSF, par la correction du front d'onde du flux lumineux incident au cours de l'exposition, sans perte de flux lumineux ni de temps d'exposition, et sans nécessiter de dispositif de mesure du front d'onde spécifiquement dédié à l'analyse des distorsions du front d'onde.

A cet effet, il est proposé un procédé d'acquisition d'une image par un imageur comportant une matrice de pixels configurés pour générer une réponse électrique lors d'une exposition à un flux lumineux incident traversant un chemin optique dans lequel est disposé un organe de correction de front d'onde, l'imageur étant adapté pour permettre une lecture non-destructrice de la réponse électrique de pixels au cours de l'exposition, le procédé comprenant les étapes suivantes :

1) début de l'exposition des pixels de la matrice de pixels au flux lumineux incident ;
2) pour une pluralité d'itérations pendant l'exposition des pixels :

2.1) lecture non-destructrice des réponses électriques de pixels d'une région d'intérêt;
2.2) détermination d'une évolution de la répartition spatiale de pixels en mode logarithmique par rapport à l'itération précédente à partir des réponses électriques des pixels de la région d'intérêt, représentative d'une fluctuation d'un front d'onde du flux lumineux incident entre cette itération et l'itération précédente ;
2.3) à partir de cette évolution, détermination d'une commande de l'organe de correction de front d'onde afin de corriger le front d'onde du flux lumineux ;
2.4) configuration de l'organe de correction de front d'onde par la commande,

3) lecture des réponses électriques des pixels de la matrice de pixels résultant en une image.

Le procédé est avantageusement complété par les caractéristiques suivantes, prises seules ou en quelconque de leurs combinaisons techniquement possibles :

- le procédé comprend une étape préalable de remise à zéro des pixels de la matrice avant l'étape 1) ;
- la détermination de l'évolution de la répartition spatiale de pixels en mode logarithmique comprend l'identification desdits pixels en mode logarithmique, ladite identification comprenant une comparaison entre chacune des réponses électriques et un seuil, le franchissement de la valeur seuil par la réponse électrique d'un pixel indiquant que ledit pixel est en mode logarithmique ;
- la détermination de l'évolution de la répartition spatiale de pixels en mode logarithmique comprend l'identification de pixels en mode logarithmique et une comparaison entre des position de ces pixels en mode logarithmique avec des positions de pixels en mode logarithmique lors de l'itération précédente ;

- la détermination de l'évolution de la répartition spatiale de pixels en mode logarithmique comprend l'identification d'un contour séparant les pixels en mode logarithmique et les pixels en mode linéaire, et la comparaison entre la position du contour et la position précédente du contour lors de l'itération précédente ;
- la commande de l'organe de correction de front d'onde est déterminée afin de stabiliser la répartition spatiale de pixels en mode logarithmique entre deux itérations ;

- la région d'intérêt regroupe moins de la moitié des pixels de la matrice ;
- le procédé comprend au moins cinq itérations mettant en œuvre les étapes 2.1) à 2.4) ;
- la région d'intérêt est définie préalablement à l'exposition des pixels ;
- le procédé comprend une étape préalable d'identification de la région d'intérêt intervenant entre l'étape 1) et l'étape 2), dans laquelle la région d'intérêt est déterminée après une étape de lecture non-destructrice des réponses électriques de l'ensemble des pixels, la région d'intérêt étant définie pour regrouper un ensemble de pixels présentant des réponses électriques en moyenne plus importantes que des réponses électriques des pixels en dehors de la région d'intérêt ;
- lors de l'étape 2.1), les réponses électriques des autres pixels hors de la région d'intérêt ne sont pas lues.

L'invention concerne également un produit programme d'ordinateur comprenant des instructions de code de programme enregistrées sur un support non transitoire utilisable dans un ordinateur pour l'exécution des étapes d'un procédé selon l'invention lorsque ledit programme est exécuté sur un ordinateur utilisant ledit support non transitoire.

L'invention concerne également un système d'acquisition d'image comprenant :

- un imageur comportant une matrice de pixels en mode photovoltaïque configurés pour générer une réponse électrique lors d'une exposition à un flux lumineux incident, l'imageur étant adapté pour permettre une lecture non-destructrice de la réponse électrique de pixels au cours de l'exposition,
- un chemin optique menant à l'imageur,
- un organe de correction de front d'onde disposé dans le chemin optique en aval de l'imageur,
- une unité de traitement de données configurée pour mettre en œuvre les étapes 2.1) à 2.4) selon l'invention.

PRESENTATION DES FIGURES

L'invention sera mieux comprise, grâce à la description ci-après, qui se rapporte à des modes de réalisations et des variantes selon la présente invention, donnés à titre d'exemples non limitatifs et expliqués avec référence aux dessins schématiques annexés, dans lesquels :

- la figure 1, déjà commentée, illustre schématiquement un système optique de l'état de la technique,
- la figure 2 illustre schématiquement un système optique selon un mode de réalisation possible de l'invention,
- la figure 3 est un diagramme montrant des étapes du procédé selon un mode de réalisation possible de l'invention,
- la figure 4 est un schéma illustrant un exemple d'un champ d'acquisition de l'imageur et une région d'intérêt,
- les figures 5a et 5b illustrent schématiquement un exemple d'évolution de la répartition spatiale de pixels en mode logarithmique entre deux itérations.

DESCRIPTION DETAILLEE

Les modes de réalisation décrits par la suite n'étant nullement limitatifs, on pourra notamment considérer des variantes de l'invention ne comprenant qu'une sélection de caractéristiques décrites ou illustrées isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection peut comprendre au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

En référence à la figure 2, le procédé d'acquisition est mis en œuvre par un système d'acquisition d'image comprenant :

- un imageur 20 comportant une matrice de pixels adapté pour permettre une lecture non-destructrice de la réponse électrique de pixels au cours de l'exposition,
- un chemin optique 21 menant à l'imageur 20,
- un organe de correction de front d'onde 22 disposé dans le chemin optique 21 en aval de l'imageur 20,
- une unité de traitement de données 25.

On entend par imageur 20 un dispositif de prise de vue capable d'acquérir une image, c'est-à-dire de rendre une information de distribution spatiale d'intensité lumineuse à partir d'un flux lumineux incident auquel est exposé l'imageur 20. A titre d'exemple non limitatif, on peut utiliser un imageur 20 offrant des possibilités similaires au capteur NSC1601T-SI de New Imaging Technologies à Verrières-le-Buisson, France, qui est un capteur InGaAs basé sur une technologie CMOS comprenant une matrice de pixels actifs avec un pas de 15 $\mu$m avec obturateur global ("global shutter"), présentant une architecture de circuit intégré de lecture (ou ROIC pour l'anglais "Readout Integrated Circuit") permettant différents modes de lecture destructrice et non-destructrice, comme par exemple ITR/IWR pour l'anglais "Integrate Then Read" (Intégrer puis lire) et Integrate While Read (Intégrer en lisant), avec une réponse spectrale de 0,9 à 1,7 $\mu$m avec une plage dynamique typique supérieure à 120 dB, pour une fréquence d'images pouvant aller jusqu'à 350 images par seconde.

L'imageur 20 comporte une matrice de pixels actifs comprenant chacun, dans leur fonction de détection et conversion du flux lumineux en signal électrique, par une photodiode utilisée en mode photovoltaïque : permettant en plus d'une réponse intrinsèque logarithmique fonction du flux lumineux incident instantané, une réponse linéaire issue de sa capacité de jonction tant que cette dernière n'est pas saturée. La matrice est adaptée pour permettre la lecture de la réponse électrique des pixels, que ceux-ci soient en mode linéaire ou logarithmique. Sous l'effet d'un flux lumineux, des porteurs de charge (paires électrons-trous) sont générés dans la jonction PN. La tension électrique aux bornes de la photodiode évolue alors d'une variation $V_D$ :

$$V_D = V_T \ln \frac{I_\lambda + I_S}{I_\lambda e^{\frac{-(I_\lambda + I_S) \times t}{V_T C_D}} + I_S}$$

avec $V_T = \frac{kT}{q}$ , k la constante de Boltzmann, T la température en K, q la charge élémentaire, t le temps d'exposition, $I_S$ le courant de saturation de la jonction de la photodiode, $I_\lambda$ le photocourant, et $C_D$ la capacité de jonction.

En fonction de la configuration du pixel, la réponse électrique peut prendre des valeurs diverses. Par exemple, les photodiodes des pixels peuvent initialement être en polarisation inverse, avec une tension initiale installée dans la photodiode lors de la remise à zéro préalablement à l'exposition. Cette tension initiale peut permettre de déterminer la plage d'utilisation de la photodiode, et plus précisément de régler le passage du mode linéaire au mode logarithmique, en fonction de la luminosité à laquelle elle est soumise et des conditions de son utilisation qui suit cette initialisation.

Lors de l'exposition, le courant photoélectrique induit dans la photodiode par les porteurs de charge déchargent progressivement la tension dans la photodiode. Au début de l'exposition, la quantité de lumière reçue étant faible, les photodiodes restent en polarisation inverse, avec des réponses électriques des pixels variant linéairement :

$$V_D = -\frac{I_\lambda \times t}{C_D}$$

Ces pixels sont dits en mode linéaire.

Lorsque l'exposition se prolonge, les photodiodes des pixels recevant un fort flux lumineux se déchargent complètement. Les pixels saturent alors. Lorsqu'un pixel sature, sa réponse électrique en tension aux bornes de sa photodiode devient une réponse logarithmique et varie selon :

$$V_D = V_T \ln \frac{I_\lambda + I_S}{I_S}$$

Ces pixels sont alors dits en mode logarithmique.

Le flux lumineux incident chemine le long du chemin optique 21 pour arriver à l'imageur 20. Contrairement au chemin optique 11 d'un système de l'état de la technique, ce chemin optique 21 n'a pas besoin de disposer d'une lame séparatrice 13 pour dévier une partie du flux lumineux vers un dispositif de mesure du front d'onde 14 dédié à l'analyse des distorsions du front d'onde. L'ensemble du flux lumineux incident, hors les différentes pertes inhérentes au passage dans le milieux de propagation et dans les optiques, peut atteindre l'imageur 20. Par conséquent, l'imageur 20 reçoit une plus grande quantité de flux lumineux. Le temps d'exposition peut ainsi être réduit, ce qui évite de nombreux problèmes.

L'organe de correction de front d'onde 22 disposé dans le chemin optique 21 en aval de l'imageur 20 corrige le front d'onde du flux lumineux incident avant que celui-ci n'atteigne l'imageur 20. L'organe de correction de front d'onde 22 est typiquement, par exemple, un microsystème électromécanique, comme un miroir déformable. Un tel miroir déformable est par exemple constitué d'une pluralité d'actionneurs sous le miroir, surface réfléchissante souple. En modifiant la position des actionneurs, on modifie la surface du miroir, et donc sa configuration. Le flux lumineux arrivant sur le miroir déformable n'est pas réfléchi de la même façon sur tout le flux, et le front d'onde s'en trouve modifié. La surface du miroir déformable peut donc être modifiée pour corriger les distorsions du front d'onde. D'autres organes de correction de front d'onde 22 sont cependant possibles, par exemple utilisant des cristaux liquides.

Afin de modifier sa configuration, l'organe de correction de front d'onde 22 reçoit une commande en provenance de l'unité de traitement de données 25. L'unité de traitement de données 25 comprend typiquement un processeur et une mémoire, et est adaptée pour recevoir des données (par exemple des mesures), les traiter, et pour envoyer des données (par exemple une commande). L'unité de traitement de données 25 est opérationnellement connectée avec l'imageur 20 et avec l'organe de correction de front d'onde 22 afin de pouvoir communiquer des données avec l'imageur 20 et l'organe de correction de front d'onde 22. L'unité de traitement de données 25 peut être composite, formée de plusieurs sous-systèmes assurant chacun une partie des fonctionnalités de l'unité de traitement de données 25. L'unité de traitement de données 25, peut être autonome avec son système embarqué ou bien disposer également d'une interface homme-machine permettant d'entrer des données et d'en afficher, comprenant par exemple un clavier et un écran.

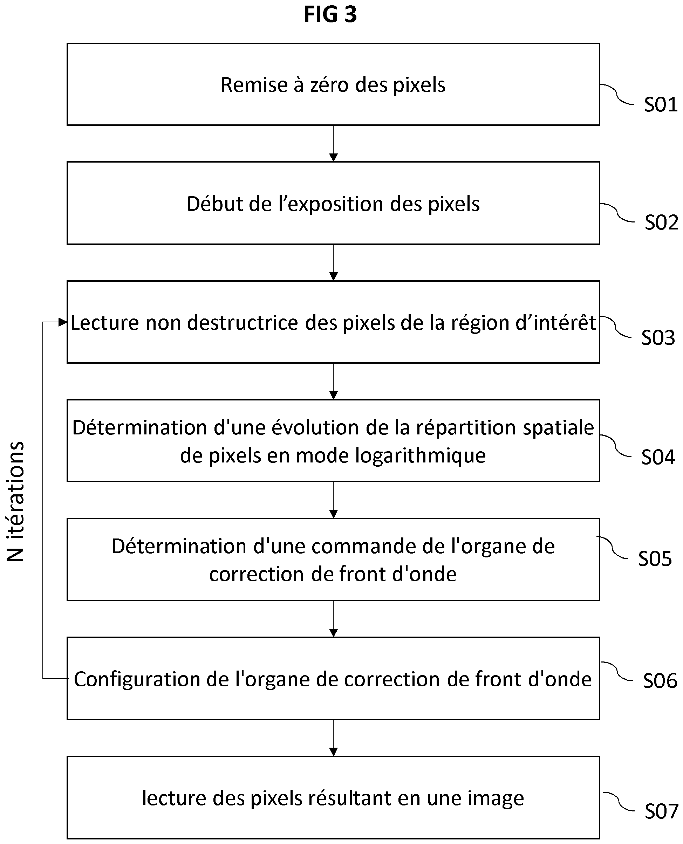

En référence à la figure 3, le procédé d'acquisition d'une image par l'imageur 20 comprend plusieurs étapes. Le procédé peut comprendre une étape préalable (S01) de remise à zéro des pixels de la matrice de pixels de l'imageur 20. Cette remise à zéro (ou "reset" en anglais) consiste à activer un signal de remise à zéro. Le signal de remise à zéro rend conducteur un transistor de remise à zéro et crée un chemin électrique imposant une tension initiale aux photodiodes des pixels. Une fois cette remise à zéro effectuée, le signal de remise à zéro est désactivé, et le chemin électrique interrompu. L'exposition des pixels de la matrice de pixels au flux lumineux incident commence alors (étape S02). Le flux lumineux parcourant le chemin optique 21 et atteignant l'imageur 20 modifie les réponses électriques des pixels, comme précédemment expliqué.

Au cours de l'exposition, le procédé met en oeuvre une pluralité d'itérations afin de corriger le front d'onde du flux lumineux incident. Au cours de chacune de ces itérations, les mêmes étapes sont répétées. La première étape (étape S03) de l'itération comprend une lecture non-destructrice des réponses électriques de pixels d'une région d'intérêt. C'est l'imageur 20 qui met en oeuvre cette lecture non-destructrice. Une lecture non-destructrice est une lecture qui permet de mesurer la réponse électrique d'un pixel sans modifier substantiellement les caractéristiques électriques du pixel. Notamment, une lecture non-destructrice n'implique ni d'évacuer les charges accumulées dans le pixel, ni de remettre à zéro le pixel (pas de reset).

Une région d'intérêt est un ensemble de pixels adjacents les uns aux autres. La région d'intérêt peut comprendre plusieurs ensembles de pixels adjacents les uns aux autres. La région d'intérêt peut regrouper tous les pixels de la matrice de l'imageur 20. Toutefois, la région d'intérêt ne regroupe de préférence qu'une partie seulement des pixels de la matrice. De préférence, la région d'intérêt regroupe moins de la moitié des pixels de la matrice, et de préférence moins de 20% des pixels de la matrice, et de préférence encore moins de 10% des pixels, voire moins de 5% des pixels de la matrice. Les réponses électriques des autres pixels hors de la région d'intérêt ne sont pas lues. Les autres pixels hors de la région d'intérêt continuent à accumuler le signal électrique issue de la conversion du flux lumineux incident, pendant les lectures non destructrices.

De préférence, le procédé comprend au moins cinq itérations, de préférence au moins dix itérations, et de préférence encore au moins vingt itérations. De fait, le plus possible d'itérations seront mises en œuvre pendant l'exposition. En effet, les itérations visent à corriger les distorsions du front d'onde, et plus il y a d'itérations pendant l'exposition, meilleure sera la correction. Il est d'ailleurs préférable que la période des itérations soit inférieure à la période de variation de ces distorsions. Dans le cas distorsions conséquences de turbulences atmosphériques, on parle alors de temps de cohérence de la turbulence atmosphérique. Le nombre d'itérations qu'il est possible de mettre en oeuvre, et leur rythme, nombre par seconde, dépend des capacités de lecture de l'imageur 20, et surtout de la taille de la région d'intérêt. Par exemple, lorsque l'imageur présente une capacité de lecture de 300 images par seconde (souvent désignée par FPS pour l'anglais "frame per second"), cela signifie qu'il peut lire un nombre de pixels équivalent à tous ses pixels 300 fois par secondes. Ainsi, dans le cas d'un imageur 20 de 320 pixels par 256 pixels (soit 81920 pixels) avec 300 FPS, l'imageur 20 peut effectuer 24576000 lectures de pixels par seconde.

En restreignant la taille de la zone lue, il est donc possible de lire cette zone plus souvent puisque moins de pixels seront lus tout en conservant la bande passante en pixel par seconde de l'imageur 20. En reprenant l'exemple ci-dessus, ne lire que les pixels d'une région d'intérêt regroupant seulement 10% des pixels de la matrice revient à lire seulement 8192 pixels à chaque lecture. Compte-tenu de la capacité de lecture de 24576000 pixels par seconde, la région d'intérêt peut être lue 3000 fois par seconde. On constate donc que la vitesse de lecture de la région d'intérêt est dix fois plus importante que la vitesse de lecture de la matrice complète. Par conséquent, le nombre d'itérations pourra être d'autant plus élevé que la région d'intérêt est petite. Dans l'exemple ci-dessus, une région d'intérêt de 10% de la matrice permet par exemple la mise en œuvre de dix fois plus d'itérations qu'avec la totalité de la matrice lue, pendant un même temps d'exposition.

Par ailleurs, le raisonnement exposé ci-dessus se place dans le cadre de la vitesse de lecture maximale de l'imageur 20. Or, l'exposition des pixels est quasiment toujours plus longue (voire beaucoup plus longue) que la période minimale permise par la capacité de lecture du capteur. En reprenant le capteur de l'exemple ci-dessus, et en supposant une durée d'exposition de 30 secondes (par exemple en astronomie), près de 90000 itérations du procédé peuvent être mises en œuvre pendant l'exposition. Dans tous les cas, le procédé comprend lors de l'acquisition d'une image de préférence au moins 10 itérations, et de préférence au moins 20 itérations, et de préférence encore au moins 100 itérations.

Il est à noter qu'une limite cependant réside dans la capacité des autres composants du système de mettre en œuvre leurs fonctions respectives pendant une itération. Toutefois, un dispositif de correction de front d'onde 22 a typiquement un période de réponse inférieure à quelques dizaines de millisecondes, et une unité de traitement de données 25 simple peut calculer beaucoup plus rapidement que requis. L'imageur 20 est donc généralement le facteur limitant.

La région d'intérêt regroupe de préférence les pixels qui reçoit la plus grande quantité de lumière au cours de l'exposition, c'est-à-dire les pixels pour lesquels le flux incident lumineux est le plus important. La région d'intérêt peut être définie préalablement à l'exposition des pixels. Par exemple, si l'utilisateur connaît à l'avance la distribution spatiale de l'intensité lumineuse dans le champ d'acquisition de l'imageur 20, il peut délimiter une région d'intérêt correspondant à la zone qui va recevoir le plus de lumière. La connaissance a priori de la distribution spatiale de l'intensité lumineuse peut par exemple venir d'une image précédemment acquise.

Alternativement, le procédé peut comprendre une étape préalable d'identification de la région d'intérêt intervenant entre le début de l'exposition et les premières itérations, dans laquelle la région d'intérêt est déterminée après une étape de lecture non-destructrice des réponses électriques de l'ensemble des pixels. La région d'intérêt est définie pour regrouper un ensemble de pixels présentant des réponses électriques en moyenne plus importantes que des réponses électriques des pixels en dehors la région d'intérêt. Dans cette région d'intérêt, sous des conditions lumineuses globalement constantes pendant l'exposition, en dehors de changements issus des corrections à effectuer, le flux lumineux plus important fera transiter les pixels plus rapidement dans la réponse logarithmique après saturation de la réponse linéaire et offrira ainsi une réponse en temps réel, échantillonnée au rythme des lectures non destructives, qui sera fonction du flux lumineux incident instantané.

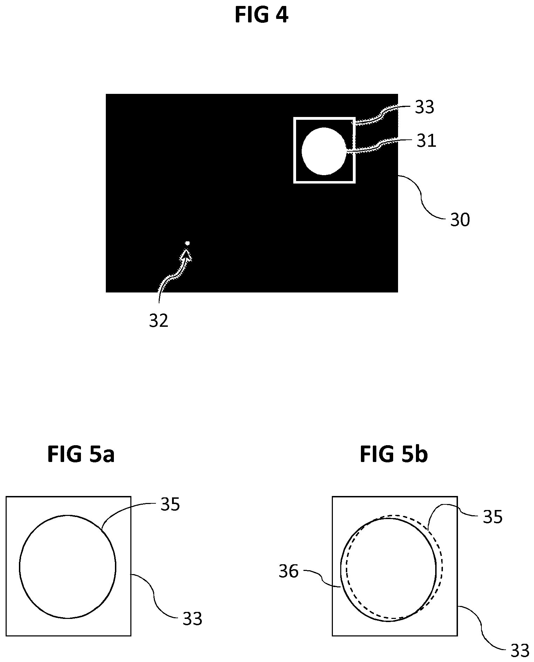

La figure 4 illustre à titre d'exemple non-limitatif un champ d'acquisition 30 d'un imageur 20 dans le domaine de l'astronomie. Dans ce champ d'acquisition 30 se trouve un premier objet 31 très lumineux, et un second objet 32 peu lumineux. Les pixels de la zone de la matrice recevant le flux lumineux du premier objet 31 reçoivent une quantité de lumière beaucoup plus importante que les pixels de la zone de la matrice recevant le flux lumineux du second objet 32. La région d'intérêt est donc choisie comme comprenant des pixels de la zone de la matrice recevant le flux lumineux du premier objet 31. De préférence, la région d'intérêt englobe tous les pixels de la zone de la matrice recevant le flux lumineux du premier objet 31. Un utilisateur acquérant cette image connaît la position du premier objet 31, et peut donc définir à l'avance la région d'intérêt 33. A défaut de connaissance a priori, il est aisé de distinguer la position du premier objet 31 grâce à sa forte intensité lumineuse dès le début de l'exposition, et donc de définir la région d'intérêt à partir d'une lecture non-destructrice de tous les pixels au début de l'exposition.

La lecture non-destructrice des réponses électriques de pixels d'une région d'intérêt permet de récolter les réponses électriques, typiquement les tensions de chacun des pixels ou leurs variations. On obtient alors, à chaque itération, une image partielle correspondant aux seuls pixels de cette région d'intérêt. A partir des réponses électriques des pixels de la région d'intérêt, une évolution de la répartition spatiale de pixels en mode logarithmique par rapport à l'itération précédente est déterminée (étape S04). Dans les faits, l'imageur 20 transmet à l'unité de traitement 25 les résultats des lectures non-destructrices, c'est-à-dire l'image partielle, et c'est unité de traitement 25 qui détermine l'évolution de la répartition spatiale de pixels en mode logarithmique.

Comme expliqué plus haut, les pixels recevant le plus de lumière saturent et passent en mode logarithmique, tandis que les autres pixels restent en mode linéaire. Dans l'exemple présenté, les pixels correspondant au premier objet 31 vont vite saturer et passer en mode logarithmique, tandis que les pixels correspondant au second objet 32 restent en mode linéaire. Alors qu'une réponse linéaire n'indique que la quantité de lumière à laquelle a été soumise un pixel depuis le début de l'exposition, une réponse logarithmique traduit en temps réel la force du flux lumineux auquel est soumis le pixel au moment de la lecture.

Ainsi, un pixel qui a été soumis à un fort flux lumineux et qui a atteint la saturation reste saturé même s'il ne reçoit plus de lumière. S'il reçoit de la lumière, sa réponse sera fonction logarithme du flux lumineux auquel le pixel est exposé au moment de la lecture. La répartition spatiale des pixels en mode logarithmique suit donc la répartition spatiale instantanée du flux lumineux auquel sont soumis les pixels. Cette répartition spatiale instantanée varie en fonction des itérations, principalement en raison des distorsions du front d'onde. Sans correction adéquate du front d'onde, cela se traduit par un déplacement de la répartition spatiale de l'intensité du flux lumineux. Sur l'exemple de la figure 4, le flux lumineux en provenance du premier objet 31 se déplace à la surface de la matrice de l'imageur 20. Ce déplacement est illustré par les figures 5a et 5b.

Sur la figure 5a, la forme 35 regroupe les pixels en mode logarithmique dans la zone d'intérêt 33 pour la lecture de l'itération n. Il s'agit des pixels recevant la lumière en provenance du premier objet 31 au moment de la lecture non-destructrice de cette itération n. Cette forme 35 correspond donc à la position du premier objet 31 perçue par l'imageur 20, c'est-à-dire la position sur l'image partielle, au moment de l'itération n. Sur la figure 5b, la forme 36 regroupe les pixels en mode logarithmique dans la zone d'intérêt 33 pour la lecture de l'itération n+1. Il s'agit des pixels recevant la lumière en provenance du premier objet 31 au moment de la lecture non-destructrice de l'itération n+1. Cette forme 36 correspond donc à la position du premier objet 31 perçue par l'imageur 20 au moment de l'itération n+1. La forme 35 est délimitée par des tirets sur la figure 5b. On constate que la forme 36 a bougé par rapport à la forme 35. Cela traduit le fait que la répartition spatiale des pixels en mode logarithmique a évolué entre l'itération n et l'itération n+1, et donc que la répartition spatiale instantanée du flux lumineux auquel sont soumis les pixels, a évolué de manière similaire entre l'itération n et l'itération n+1. Ainsi, la position du premier objet 31 perçue par l'imageur 20 a évolué entre l'itération n et l'itération n+1. De même, le premier objet 31 perçu par l'imageur 20 peut présenter des distorsions variant avec les itérations, qui se traduisent également par une évolution de la répartition spatiale instantanée du flux lumineux.

Or, cette évolution de la répartition spatiale instantanée du flux lumineux est principalement due aux distorsions du front d'onde par la turbulence atmosphérique ou à tout autre variation, vibrations par exemple, dans le chemin optique 21 entre deux itérations. Par conséquent, l'évolution de la répartition spatiale des pixels en mode logarithmique est représentative d'une fluctuation d'un front d'onde du flux lumineux incident entre l'itération n et l'itération n+1. Une commande de l'organe de correction de front d'onde 22 peut être déterminée (étape S05) par l'unité de traitement 25 qui reçoit les résultats des lectures non-destructrices en provenance de l'imageur 20 afin de compenser cette évolution de la répartition spatiale, c'est-à-dire afin de compenser le déplacement du contour entre l'itération n et l'itération n+1.

Dans le détail, la détermination de l'évolution de la répartition spatiale de pixels en mode logarithmique peut comprendre l'identification desdits pixels en mode logarithmique parmi les pixels de la zone d'intérêt dont les réponses ont été lues au cours de l'itération. Pour ce faire, l'identification peut comprendre une comparaison entre chacune des réponses électriques de ces pixels et un seuil, le franchissement de la valeur seuil par la réponse électrique d'un pixel indiquant que ledit pixel est en mode logarithmique. Comme expliqué précédemment, un pixel ne passe en mode logarithmique que lorsqu'il devient saturé. Avant cette saturation, la réponse électrique est linéaire et croît avec l'exposition au flux jusqu'à une valeur de saturation. En plaçant le seuil légèrement au-dessus de cette valeur de saturation, il est possible discriminer les pixels en mode logarithmique des pixels en mode linéaire. L'identification des pixels en mode logarithmique à chaque itération permet de déterminer l'évolution de leur répartition spatiale, et donc de déterminer une commande de l'organe de correction de front d'onde 22.

La détermination de l'évolution de la répartition spatiale de pixels en mode logarithmique peut alors comprendre l'identification de pixels en mode logarithmique et une comparaison entre des position de ces pixels en mode logarithmique avec des positions de pixels en mode logarithmique lors de l'itération précédente. Toutes les positions des pixels en mode logarithmique peuvent être utilisées lors de la comparaison. La comparaison peut également être restreinte à une partie seulement des pixels en mode logarithmique, dont les positions présentent un intérêt accru. C'est notamment le cas pour les pixels appartenant à un contour séparant des pixels en mode logarithmique et des pixels en mode linéaire.

De fait, la détermination de l'évolution de la répartition spatiale de pixels en mode logarithmique peut comprendre l'identification d'un contour séparant les pixels en mode logarithmique et les pixels en mode linéaire, notamment après l'identification des pixels en mode logarithmique. Sur la figure 5a, ce contour correspond au bord de la forme 35, et au bord de la forme 36 sur la figure 5b. La détermination de l'évolution de la répartition spatiale de pixels en mode logarithmique peut alors comprendre la comparaison entre la position du contour à une itération (par exemple l'itération n) et la position précédente du contour lors de l'itération précédente (par exemple l'itération n+1), qui correspond à l'évolution de la répartition spatiale des pixels en mode logarithmique permettant de déterminer la commande.

S'agissant d'une image partielle (restreinte à la région d'intérêt), il est possible d'utiliser divers procédés de détection et de segmentation utilisés en imagerie, comme par exemple, une simple sélection de la réponse de la totalité ou d'une partie des pixels de l'image partielle de l'itération précédente dont la luminance se situe dans la réponse logarithmique de l'imageur 20. On obtient alors un motif spatial. Cette sélection est alors suivie d'une recherche de ce motif spatial dans l'image de l'itération courante. Des méthodes de segmentation plus sophistiqués incluant une connaissance à priori de la forme de l'objet à détecter peuvent être envisagées (comme par exemple un disque dans le cas d'un premier objet 31 correspondant à une étoile). Ces méthodes peuvent considérer l'ensemble des pixels de la zone d'intérêt ou seulement ceux dont la luminance se situe dans la réponse logarithmique du capteur.

Dans tous les cas, la commande de l'organe de correction de front d'onde est déterminée afin de stabiliser la répartition spatiale de pixels en mode logarithmique entre deux itérations qui se suivent. Cette commande, déterminée par l'unité de traitement 25, est envoyée à l'organe de correction de front d'onde 22. La configuration de l'organe de correction de front d'onde 22 est modifiée en fonction de la commande (étape S06). Par exemple, si l'organe de correction de front d'onde 22 est un miroir déformable, les orientations des différents éléments miroir sont modifiées pour corriger le front d'onde.

Ce processus est réitéré pour chaque itération (il peut ne pas y avoir de comparaison lors de la première itération), afin que la correction suive les variations des distorsions du front d'onde. Ainsi, le flux lumineux arrivant sur l'imageur 20 est en permanence corrigé pendant l'exposition, sur la base de l'exploitation de la région d'intérêt 33. A la fin du procédé, lorsque l'exposition a été jugée suffisante, et que plusieurs itérations ont été effectuées pendant l'exposition, les réponses électriques des pixels de la matrice de pixels sont lues (étape S07) résultant en une image. En reprenant l'exemple de la figure 4, l'exploitation des pixels de la région d'intérêt 33, saturés par le premier objet 31, permet d'acquérir une image comportant une représentation de très grande qualité de l'objet 32, le flux lumineux ayant été corrigé en permanence et permettant dans les zones de l'image encore en réponse linéaire une augmentation sensible du rapport signal sur bruit et de la résolution dus à l'amélioration de la PSF pendant l'accumulation du signal durant l'exposition entre deux remises à zéro (ou reset) des pixels de l'imageur.

L'invention n'est pas limitée au mode de réalisation décrit et représenté aux figures annexées. Des modifications restent possibles, notamment du point de vue de la constitution des diverses caractéristiques techniques ou par substitution d'équivalents techniques, sans sortir pour autant du domaine de protection de l'invention.

## Revendications

1. Procédé d'acquisition d'une image par un imageur (20) comportant une matrice de pixels configurés pour générer une réponse électrique lors d'une exposition à un flux lumineux incident traversant un chemin optique (21) dans lequel est disposé un organe de correction de front d'onde (22), l'imageur (20) étant adapté pour permettre une lecture non-destructrice de la réponse électrique de pixels au cours de l'exposition, le procédé comprenant les étapes suivantes :

   1) début (S02) de l'exposition des pixels de la matrice de pixels au flux lumineux incident ;
   2) pour une pluralité d'itérations pendant l'exposition des pixels :

      2.1) lecture (S03) non-destructrice des réponses électriques de pixels d'une région d'intérêt (33) ;
      2.2) détermination (S04) d'une évolution de la répartition spatiale de pixels en mode logarithmique par rapport à l'itération précédente à partir des réponses électriques des pixels de la région d'intérêt (33), représentative d'une fluctuation d'un front d'onde du flux lumineux incident entre cette itération et l'itération précédente ;
      2.3) à partir de cette évolution, détermination (S05) d'une commande de l'organe de correction de front d'onde (22) afin de corriger le front d'onde du flux lumineux ;
      2.4) configuration (S06) de l'organe de correction de front d'onde par la commande,

   3) lecture (S07) des réponses électriques des pixels de la matrice de pixels résultant en une image.

2. Procédé selon la revendication 1, comprenant une étape préalable de remise à zéro des pixels de la matrice avant l'étape 1).

3. Procédé selon l'une des revendications 1 à 2, dans lequel la détermination de l'évolution de la répartition spatiale de pixels en mode logarithmique comprend l'identification desdits pixels en mode logarithmique, ladite identification comprenant une comparaison entre chacune des réponses électriques et un seuil, le franchissement de la valeur seuil par la réponse électrique d'un pixel indiquant que ledit pixel est en mode logarithmique.

4. Procédé selon l'une des revendications 1 à 3, dans lequel la détermination de l'évolution de la répartition spatiale de pixels en mode logarithmique comprend l'identification de pixels en mode logarithmique et une comparaison entre des positions de ces pixels en mode logarithmique avec des positions de pixels en mode logarithmique lors de l'itération précédente

5. Procédé selon la revendication 4, dans lequel la détermination de l'évolution de la répartition spatiale de pixels en mode logarithmique comprend l'identification d'un contour séparant les pixels en mode logarithmique et les pixels en mode linéaire, et la comparaison entre la position du contour et la position précédente du contour lors de l'itération précédente.

6. Procédé selon l'une des revendications 1 à 5, dans lequel la commande de l'organe de correction de front d'onde est déterminée afin de stabiliser la répartition spatiale de pixels en mode logarithmique entre deux itérations.

7. Procédé selon l'une des revendications 1 à 6, dans lequel la région d'intérêt regroupe moins de la moitié des pixels de la matrice.

8. Procédé selon l'une des revendications 1 à 7, dans lequel le procédé comprend au moins cinq itérations mettant en œuvre les étapes 2.1) à 2.4).

9. Procédé selon l'une des revendications 1 à 8, dans lequel la région d'intérêt est définie préalablement à l'exposition des pixels.

10. Procédé selon l'une des revendications 1 à 8, comprenant une étape préalable d'identification de la région d'intérêt intervenant entre l'étape 1) et l'étape 2), dans laquelle la région d'intérêt est déterminée après une étape de lecture non-destructrice des réponses électriques de l'ensemble des pixels, la région d'intérêt étant définie pour regrouper un ensemble de pixels présentant des réponses électriques en moyenne plus importantes que des réponses électriques des pixels en dehors la région d'intérêt.

11. Procédé selon l'une des revendications 1 à 10, dans lequel lors de l'étape 2.1), les réponses électriques des autres pixels hors de la région d'intérêt ne sont pas lues.

12. Produit programme d'ordinateur comprenant des instructions de code de programme enregistrées sur un support non transitoire utilisable dans un ordinateur pour l'exécution des étapes d'un procédé selon l'une des revendications précédentes lorsque ledit programme est exécuté sur un ordinateur utilisant ledit support non transitoire.

**13.** Système d'acquisition d'image comprenant :

- un imageur (20) comportant une matrice de pixels en mode photovoltaïque configurés pour générer une réponse électrique lors d'une exposition à un flux lumineux incident, l'imageur étant adapté pour permettre une lecture non-destructrice de la réponse électrique de pixels au cours de l'exposition,
- un chemin optique (21) menant à l'imageur,
- un organe de correction de front d'onde (22) disposé dans le chemin optique en aval de l'imageur,
- une unité de traitement de données (25) configurée pour mettre en œuvre les étapes 2.1) à 2.4) selon l'une quelconque des revendications 1 à 11.

**Patentansprüche**

**1.** Verfahren zur Aufnahme eines Bildes durch einen Bildaufnehmer (20), der eine Matrix von Pixeln umfasst, die dazu ausgestaltet ist, bei einer Belichtung mit einem einfallenden Lichtstrom, der einen Lichtweg (21) durchquert, in dem ein Wellenfront-Korrekturorgan (22) angeordnet ist, eine elektrische Reaktion zu erzeugen, wobei der Bildaufnehmer (20) dazu angepasst ist, ein zerstörungsfreies Ablesen der elektrischen Reaktion von Pixeln im Laufe der Belichtung zu ermöglichen, wobei das Verfahren die folgenden Schritte umfasst:

1) Beginnen (S02) der Belichtung der Pixel der Matrix von Pixeln mit dem einfallenden Lichtstrom;
2) während einer Vielzahl von Iterationen während der Belichtung der Pixel:

2.1) zerstörungsfreies Ablesen (S03) der elektrischen Reaktionen von Pixeln einer Region von Interesse (33);
2.2) Bestimmen (S04) einer Entwicklung der räumlichen Verteilung von Pixeln im logarithmischen Modus in Bezug auf die vorhergehende Iteration ausgehend von den elektrischen Reaktionen der Pixel der Region von Interesse (33), die für eine Schwankung einer Wellenfront des einfallenden Lichtstroms zwischen dieser Iteration und der vorhergehenden Iteration repräsentativ ist;
2.3) ausgehend von dieser Entwicklung, Bestimmen (S05) einer Steuerung des Wellenfront-Korrekturorgans (22), um die Wellenfront des Lichtstroms zu korrigieren;
2.4) Ausgestalten (S06) des Wellenfront-Korrekturorgans durch die Steuerung,

3) Ablesen (S07) der elektrischen Reaktionen der Pixel der Matrix von Pixeln, die ein Bild ergeben.

**2.** Verfahren nach Anspruch 1, das einen vorhergehenden Schritt des Nullstellens der Pixel der Matrix vor dem Schritt 1) umfasst.

**3.** Verfahren nach einem der Ansprüche 1 bis 2, wobei das Bestimmen der Entwicklung der räumlichen Verteilung von Pixeln im logarithmischen Modus das Identifizieren der Pixel im logarithmischen Modus umfasst, wobei das Identifizieren ein Vergleichen zwischen jeder der elektrischen Reaktionen und einem Schwellenwert umfasst, wobei das Überschreiten des Schwellenwerts durch die elektrische Reaktion eines Pixels angibt, dass das Pixel sich im logarithmischen Modus befindet.

**4.** Verfahren nach einem der Ansprüche 1 bis 3, wobei das Bestimmen der Entwicklung der räumlichen Verteilung von Pixeln im logarithmischen Modus das Identifizieren von Pixeln im logarithmischen Modus und ein Vergleichen zwischen Positionen dieser Pixel im logarithmischen Modus und Positionen von Pixeln im logarithmischen Modus bei der vorhergehenden Iteration umfasst.

**5.** Verfahren nach Anspruch 4, wobei das Bestimmen der Entwicklung der räumlichen Verteilung von Pixeln im logarithmischen Modus das Identifizieren eines Umrisses, der die Pixel im logarithmischen Modus und die Pixel im linearen Modus trennt, und das Vergleichen zwischen der Position des Umrisses und der vorhergehenden Position des Umrisses bei der vorhergehenden Iteration umfasst.

**6.** Verfahren nach einem der Ansprüche 1 bis 5, wobei die Steuerung des Wellenfront-Korrekturorgans bestimmt wird, um die räumliche Verteilung von Pixeln im logarithmischen Modus zwischen zwei Iterationen zu stabilisieren.

**7.** Verfahren nach einem der Ansprüche 1 bis 6, wobei die Region von Interesse weniger als die Hälfte der Pixel der Matrix zusammenfasst.

**8.** Verfahren nach einem der Ansprüche 1 bis 7, wobei das Verfahren mindestens fünf Iterationen umfasst, welche die Schritte 2.1) bis 2.4) durchführen.

**9.** Verfahren nach einem der Ansprüche 1 bis 8, wobei die Region von Interesse vor der Belichtung der Pixel definiert wird.

**10.** Verfahren nach einem der Ansprüche 1 bis 8, das einen vorhergehenden Schritt zum Identifizieren der Region von Interesse umfasst, der zwischen dem Schritt 1) und dem Schritt 2) erfolgt und in dem die Region von Interesse nach einem Schritt des zerstörungsfreien Ablesens der elektrischen Reaktionen der Gesamtheit der Pixel bestimmt wird, wobei die Region von Interesse definiert wird, um eine Gesamtheit von Pixeln zusammenzufassen, die elektrische Reaktionen aufweisen, die im Durchschnitt größer sind als elektrische Reaktionen der Pixel außerhalb der Region von Interesse.

11. Verfahren nach einem der Ansprüche 1 bis 10, wobei im Schritt 2.1) die elektrischen Reaktionen der anderen Pixel außerhalb der Region von Interesse nicht abgelesen werden.

12. Computerprogrammprodukt, das Programmcodeanweisungen umfasst, die auf einem nichtflüchtigen Datenträger gespeichert sind, der in einem Computer zur Ausführung der Schritte eines Verfahrens nach einem der vorhergehenden Ansprüche verwendbar ist, wenn das Programm auf einem Computer unter Verwendung des nichtflüchtigen Datenträgers ausgeführt wird.

13. Bildaufnahmesystem, das Folgendes umfasst:

- einen Bildaufnehmer (20), der eine Matrix von Pixeln im photovoltaischen Modus umfasst, die dazu ausgestaltet sind, bei einer Belichtung mit einem einfallenden Lichtstrom eine elektrische Reaktion zu erzeugen, wobei der Bildaufnehmer dazu angepasst ist, ein zerstörungsfreies Ablesen der elektrischen Reaktion von Pixeln im Laufe der Belichtung zu ermöglichen,
- einen Lichtweg (21), der zu dem Bildaufnehmer führt,
- ein Wellenfront-Korrekturorgan (22), das in dem Lichtweg dem Bildaufnehmer nachgeschaltet angeordnet ist,
- eine Datenverarbeitungseinheit (25), die dazu ausgestaltet ist, die Schritte 2.1) bis 2.4) nach einem der Ansprüche 1 bis 11 durchzuführen.

**Claims**

1. A method of acquiring an image by an imager (20) having an array of pixels configured to generate an electrical response upon exposure to incident light flux passing through an optical path (21) in which a wavefront correction device (22) is disposed, the imager (20) being adapted to allow non-destructive readout of the electrical response of pixels during exposure, the method comprising the following steps:

1) beginning (S02) of the exposure of the pixels of the pixel array to the incident light flux;
2) for a plurality of iterations during the exposure of the pixels:

2.1 ) non-destructive reading (S03) of the electrical responses of pixels of a region of interest (33) ;
2.2) determination (S04) of an evolution of the spatial distribution of pixels in logarithmic mode with respect to the preceding iteration from the electrical responses of the pixels of the region of interest (33), representative of a fluctuation of a wavefront of the incident light flux between this iteration and the preceding iteration
2.3) from this evolution, determination (S05) of a control of the wavefront correction device (22) in order to correct the wavefront of the light flux;
2.4) configuration (S06) of the wavefront correction device by the control,

3) reading (S07) the electrical responses of the pixels of the pixel array resulting in an image.

2. The method of claim 1, comprising a prior step of resetting the pixels of the array prior to step 1).

3. The method according to any of claims 1 to 2, wherein determining the evolution of the spatial distribution of pixels in logarithmic mode comprises identifying said pixels in logarithmic mode, said identification comprising a comparison between each of the electrical responses and a threshold, the crossing of the threshold value by the electrical response of a pixel indicating that said pixel is in logarithmic mode.

4. The method of any one of claims 1 to 3, wherein determining the spatial distribution evolution of pixels in logarithmic mode comprises identifying pixels in logarithmic mode and comparing positions of such pixels in logarithmic mode with positions of pixels in logarithmic mode in the previous iteration

5. The method of claim 4, wherein determining the change in spatial distribution of log mode pixels comprises identifying a contour separating the pixels in logarithmic mode and the pixels in linear mode, and comparing the position of the contour with the previous position of the contour in the previous iteration.

6. The method of any of claims 1-5, wherein the control of the wavefront correction device is determined to stabilize the spatial distribution of pixels in logarithmic mode between two iterations.

7. The method of any of claims 1 to 6, wherein the region of interest aggregates less than half of the pixels in the array.

8. The method of any of claims 1 to 7, wherein the method comprises at least five iterations implementing steps 2.1) to 2.4).

9. The method of any of claims 1 to 8, wherein the region of interest is defined prior to the exposure of the pixels.

10. Method according to one of claims 1 to 8, comprising a prior step of identifying the region of interest occurring between step 1) and step 2), in which the region of interest is determined after a step of non-destructive reading of the electrical responses of the set of pixels, the region of interest being defined so as to group together a set of pixels exhibiting electrical responses that are on average greater than the electrical responses of pixels outside the region of interest.

11. The method of any of claims 1 to 10, wherein in step 2.1), the electrical responses of other pixels outside the region of interest are not read.

12. A computer program product comprising program code instructions stored on a non-transitory medium usable in a computer for performing the steps of a method according to any of the preceding claims when said program is executed on a computer using said non-transitory medium.

13. An image acquisition system comprising:

- an imager (20) having an array of pixels in photovoltaic mode configured to generate an electrical response upon exposure to incident light flux, the imager being adapted to allow non-destructive reading of the electrical response of pixels during exposure,
- an optical path (21) leading to the imager
- a wavefront correction device (22) disposed in the optical path downstream of the imager
- a data processing unit (25) configured to implement steps 2.1) to 2.4) according to any of claims 1 to 11.

## FIG 1
### (Art antérieur)

12

15

Unité de traitement

11

13

14

10

## FIG 2

22

25

Unité de traitement

21

20

## FIG 3

| Remise à zéro des pixels | S01 |

| Début de l'exposition des pixels | S02 |

| Lecture non destructrice des pixels de la région d'intérêt | S03 |

| Détermination d'une évolution de la répartition spatiale de pixels en mode logarithmique | S04 |

| Détermination d'une commande de l'organe de correction de front d'onde | S05 |

| Configuration de l'organe de correction de front d'onde | S06 |

| lecture des pixels résultant en une image | S07 |

N itérations

**FIG 4**

33

31

30

32

**FIG 5a**

35

33

**FIG 5b**

35

36

33

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20050098707 A1